# FASCICULE DE BREVET EUROPEEN

(11) **EP 0 851 028 B1**
(45) Date de publication et mention de la délivrance du brevet: **06.12.2006**
(21) Numéro de dépôt: 96203707.3
(22) Date de dépôt: 24.12.1996
(51) Int. Cl.: C12N 5/10, G01N 33/50, C12Q 1/68, A61K 35/44

(54) **Lignée immortalisée de cellules épithéliales humaines de la cornée**
Immortalisierte menschliche Epithelzell-Linie aus Kornea
Immortalized human epithelial cell line from cornea

(43) Date de publication de la demande: 01.07.1998
(73) Titulaire: SOCIETE DES PRODUITS NESTLE S.A., 1800 Vevey (CH)
(72) Inventeur: Offord Cavin, Elizabeth, 1041 Poliez-Pittet (CH); Tromvoukis, Yvonne, 1074 Mollie-Margot (CH); Pfeifer, Andrea M.A., 1806 St-Legier (CH); Sharif, Naj, Arlington, TX76015 (US)
(74) Mandataire: Chautard, Cécile

(56) Documents cités:
- US-A- 5 585 265
- INVEST OPHTHALMOL VISUAL SCI 34 (9). 1993. 2665-2671, XP000674634 ARAKI K ET AL: "IMMORTALIZATION OF RABBIT CORNEAL EPITHELIAL CELLS BY A RECOMBINANT SV40-ADENOVIRUS VECTOR."
- INVESTIGATIVE OPHTHALMOLOGY & VISUAL SCIENCE 36 (3). 1995. 614-621, XP000674633 ARAKI-SASAKI K ET AL: "An SV40- immortalized human corneal epithelial cell line and its characterization."
- INVESTIGATIVE OPHTHALMOLOGY & VISUAL SCIENCE 34 (12). 1993. 3429-3441, XP000674621 KAHN C R ET AL: "Human corneal epithelial primary cultures and cell lines with extended life span: In vitro model for ocular studies."
- DNA AND CELL BIOLOGY, vol. 13, no. 9, 1994, pages 909-921, XP000615992 DUTT K. ET AL.: "Establishment of Human Retinal Cell Line by Transfection of SV40 T Antigen Gene with Potential to Undergo Neuronal Differentiation"

## Description

La présente invention a pour objet une nouvelle lignée immortalisée de cellules épithéliales humaines de la cornée, ainsi que son utilisation dans des procédés d'identification d'agents mutagéniques, toxiques, ou bénéfiques pour le métabolisme des cellules de la cornée.

### Etat de la technique

Depuis de nombreuses années, des efforts sont entrepris pour développer des lignées cellulaires humaines adaptées à l'étude des maladies humaines, comme les infections, les inflammations ou les cancers, par exemple. Parmi les cellules impliquées souvent dans l'apparition de maladies, on peut compter les cellules épithéliales qui sont sensibles à l'environnement du corps humain.

Les cellules épithéliales se distinguent des autres cellules du corps humain par l'expression de composés ou structures retrouvés uniquement dans les cellules épithéliales, comme par exemple les cytokeratines (Moll et al., Cell, 31, 11-24, 1982), les connections entre les cellules (Gumbiner et al., Cell, 69, 385-387, 1992), et la vimentine (Richard et al., Arch. Dematol. Res., 282, 512-515, 1990).

D'autres composés sont également retrouvés généralement dans les cellules épithéliales humaines, mais pas uniquement dans les cellules épithéliales, comme les cytochromes P450 (Mercurio et al., Biochem. Biophy. Research Communications, 210, n°2, 350-355, 1995; McKinnon et al., Gut, 36, 259-267, 1995), les enzymes impliquées dans la défense contre l'oxydation cellulaire (Cu/Zn-superoxide dismutase, glutathion péroxidase, glutathion réductase et catalase: Albers et al., Toxicology and Applied Pharmacology, 109, 507-513, 1991) et/ou dans la détoxification d'électrophiles (glutathion-S-transférases α, µ ou π: Singh et al. Exp. Eye Res., 40, 431-437, 1985; l'aldehyde réductase: Ellis et al., Biochemical J., 312, 535-541, 1995)

Les cellules épithéliales de la cornée humaine se distinguent également des autres cellules épithéliales humaines par l'expression de composés qui sont retrouvés uniquement dans les cellules épithéliales de la cornée, comme par exemple la cytokératine de 64kD et la glutathion-S-transférase hGST 5.8 retrouvées dans les tissus oculaires (hGST 5.8: Singhal et al., Invest. Ophthalmol. Vis. Sci., 36, 142-150, 1995; 64kD: Kiritoshi et al., Invest. Ophthalmol. Vis. Sci., 32, 3073-3077, 1991).

L'analyse de l'expression de certaines cytokines et facteurs de croissance dans le cellules épithéliales de cornée humaine, sans stimulation inflammatoire, a déjà été déterminée par Cubitt et al, Wilson et al., Kennedy et al. et De-Quan et al. (J. Cellular Physiol, 163, 61-79, 1995; J. Clinical Investigation, 95, 82-88, 1995; Invest. Ophtha. & Visual Sci., 33, 1756-1762, 1992; Invest. Ophtha. & Visual Sci., 36, 330-340, 1995; Invest. Ophtha. & Visual Sci., 34, 3199-3210, 1993). Par ailleurs, Rosenbaum et al. ont suggéré que le profile en cytokines des cellules de la cornée pourrait être un moyen pour détecter efficacement la présence de certaines maladies de la cornée (Invest. Ophthalmol. Vis. Sci., 36, 2151-2155, 1995). Le profile d'expression de certaines cytokines et facteurs de croissance dans les cellules épithéliales de la cornée permet donc de caractériser et de différencier les cellules épithéliales de cornée normales, des autres cellules, et en particulier des cellules épithéliales de cornées anormales.

Par ailleurs, les méthodes de criblage de molécules non-inflammatoires pour les yeux font appels à des animaux de laboratoire. Pour remédier au fait qu'il subsiste des différences sensibles, morphologiques et biochimiques, entre les yeux humains et ceux de ces animaux, Hainsworth et al. proposent de réaliser ces tests de criblage sur des cultures de cellules épithéliales primaires de cornée (J. Tissue Culture Meth., 13, 45-48, 1991). Malheureusement, les cultures de cellules primaires de cornée cessent de se multiplier après un ou deux passages, chaque passage consistant à repiquer les cellules dans du milieu frais après croissance jusqu'à confluence. De plus, ces cellules primaires ne survivent pas à une congélation dans de l'azote liquide.

Pour pallier ces inconvénients, des lignées de cellules épithéliales humaines de cornée ont été développées par Kahn et al. (Investigative Opht. & Visual Sci., 34, 3429-3441, 1993) et Araki-Sasaki et al. (Investigative Opht. & Visual Sci., 36, 614-621, 1995). Malheureusement, ces lignées ne sont pas encore pleinement satisfaisantes.

En effet, les lignées développées par Kahn et al. ne sont pas complètement immortalisées, car elles conservent certaines caractéristiques de différentiation originelles jusqu'à environ 25 passages successifs de culture (voir Kahn et al.). Ces lignées libèrent aussi des virus SV40 dans le milieu de culture (voir l'analyse de Araki-Sasaki et al.). Enfin, on ne sait pas si elles expriment effectivement d'autres marqueurs de différentiation, tels que la glutathion-S-transférase hGST5.8, et un profile approprié en cytokines et en facteurs de croissance, ainsi que des marqueurs spécifiques ou nécessaires à une réaction inflammatoire. En outre, US 5,585,265 (C. R. Kahn, J. Rhim) décrit des lignées de cellules épithéliales humaines de cornée.

Les lignées immortalisées développées par Araki-Sasaki et al. sont par contre incapables de se stratifier dans des essais de reconstruction de cornée *in-vivo* (voir Araki-Sasaki et al.), et on ne sait pas si elles expriment certains marqueurs de différentiation, tels que la glutathion-S-transférase hGST5.8, et un profile approprié en cytokines et en facteurs de croissance, ainsi que des marqueurs spécifiques ou nécessaires à une réaction inflammatoire

Le but de l'invention vise à fournir une nouvelle lignée de cellules épithéliales humaines de la cornée qui se rapproche génétiquement et physiologiquement des cellules épithéliales normales de la cornée humaine, au point qu'elle peut être utilisée efficacement dans des essais de criblage de molécules potentiellement inflammatoires.

### Résumé de l'invention

A cet effet, l'invention concerne la lignée immortalisées de cellules épithéliales humaines de cornée présentant le numéro de dépôt CNCM I-1777

Un autre aspect de l'invention concerne un nouveau procédé pour identifier l'effet mutagène, toxique ou bénéfique d'un agent sur le métabolisme des cellules de la cornée, dans lequel (1) on fait réagir une culture comprenant une lignée cellulaire selon l'invention avec un agent soupçonné d'être un agent mutagène, toxique ou bénéfique pour le métabolisme des cellules de la cornée humaine, et (2) on détermine les effets dudit agent sur ladite lignée cellulaire.

L'invention concerne aussi un kit de diagnostique comprenant les cellules épithéliales immortalisées de la cornée humaine selon l'invention, et des réactifs pour déterminer une réponse métaboliques desdites cellules à des agents mutagéniques, toxiques ou bénéfiques pour lesdites cellules.

Enfin, l'invention a aussi pour objet toutes utilisations de la lignée cellulaire selon l'invention, dans des procédés d'identification d'agents mutagéniques, toxiques ou bénéfiques pour le métabolisme des cellules de la cornée, ainsi que toutes utilisations de ces lignées en tant qu'agent pharmaceutique actif.

### Description des figures

Figure 1: représentation schématique du plasmide pZIPSVU19 qui a été utilisé pour préparer le vecteur p1/SV40U19 destiné à immortaliser les cellules épithéliales primaires de la cornée humaine
Figure 2: pourcentage d'accumulation de [3H] phosphoinositides par des cellules épithéliales primaires de cornée humaine et par les cellules CNCM, en fonction de la concentration de bradykinine appliquée.
Figure 3: pourcentage d'accumulation de [3H] phosphoinositides par des cellules épithéliales primaires de cornée humaine et par les cellules CNCM, en fonction de la concentration d'histamine appliquée.
Figure 4: pourcentage d'accumulation de [3H] phosphoinositides par des cellules épithéliales primaires de cornée humaine et par les cellules CNCM, en fonction de la concentration de PAF appliquée.

### Description détaillée de l'invention

Dans le cadre de la présente invention, les expressions "cellules normales", "cellules primaires" ou "cellules non-immortalisées" désignent des cellules épithéliales de la cornée humaine que l'on peut prélever sur la cornée d'un adulte sain ne présentant pas de déficiences physiologiques ou génétiques handicapantes, et que l'on peut cultiver pendant un temps limité sans qu'elles perdent leurs caractéristiques de différenciation originelle.

Par contre, l'expression "cellules immortalisées" désigne des cellules qui ont subi une manipulation génétique, par l'intermédiaire d'une construction d'ADN, qui les rend capables de se multiplier indéfiniment, c'est à dire à plus de 25 passages, de préférence au moins 60 passages.

De même, le mot "passage" désigne le processus consistant à prendre un aliquote d'une culture confluante ou à saturation d'une lignée cellulaire, à ensemencer un milieu frais, et à cultiver la lignée jusqu'à confluence ou saturation. Les lignées cellulaires sont ainsi cultivées traditionnellement par passages successifs dans des milieux frais. Il faut noter que les lignées cellulaires peuvent perdre leurs caractéristiques de différentiation originelle après plusieurs passages successifs. Il est donc extrêmement avantageux de pouvoir disposer d'une lignée dont ses caractéristiques sont conservées, même après de nombreux passages, de préférence au moins 30 à 60 passages.

Enfin, l'expression "caractéristiques de différentiation originelle" désigne à la fois les marqueurs retrouvés spécifiquement sur les cellules épithéliales humaines et les marqueurs de différentiation retrouvés spécifiquement sur les cellules épithéliales de la cornée humaine.

La lignée immortalisée de cellules épithéliales de la cornée humaine selon l'invention n'exprime pas de marqueurs tumoraux, c'est à dire ne présente pas de gènes cancérigènes ou n'expriment pas d'ARN messagers (ARNm), protéines, et/ou structure cellulaires différentiées caractéristiques de la transformation des cellules en cellules tumorales. On peut détecter la présence de ces marqueurs par hybridation ou PCR de leur ADN avec une sonde spécifique, par des anticorps, et/ou par microscopie électronique, par exemple. La présence d'un marqueur dans une cellule ne signifie pas que ladite cellule est susceptible de conférer un cancer après quelques mois à une souris sans défense immunitaire, mais traduit plutôt un état transformé cancéreux des cellules par rapport aux cellules originelles dont elles sont issues.

La lignée selon l'invention est aussi dépourvue de virus, c'est à dire elle est incapables de produire les virus qui les ont initialement immortalisés, comme c'est le cas pour les lignées développées par Kahn et al. (voir l'introduction de Araki-Sasaki et al.).

La lignée selon l'invention est capable de se stratifier, c'est à dire capables de s'organiser en strates successives. Pour cela, il suffit de cultiver jusqu'à confluence la lignée selon l'invention dans un milieu sans sérum comprenant 1,5 mM de CaCl, puis d'observer visuellement si les cellules se développent en strates, par exemple.

La lignée selon l'invention exprime par contre des marqueurs métaboliques spécifiques de cellules épithéliales humaines normales, c'est à dire des marqueurs retrouvées généralement dans les cellules épithéliales.

Ces marqueurs spécifiques peuvent être un ARN messager (ARNm), une protéine, et/ou une structure cellulaire différentiée pouvant provenir de cellules épithéliales de la peau, de l'oeil, du tractus intestinal ou du foie, par exemple. Les cellules épithéliales humaines selon l'invention expriment au moins deux marqueurs choisis dans le groupe formé par la vimentine, les cytokératines, les connections entre les cellules (appelées encore en anglais "tight junctions"), les cytochromes P450, la glutathion-S-transférase (GST), la Cu/Zn-superoxide dismutase (SOD), la glutathion péroxidase (GP), la glutathion réductase (GR), la catalase (CA), et l'aldehyde réductase (AR).

On peut aussi noter que la lignée selon l'invention peut exprimer des enzymes impliquées dans l'oxydation cellulaire (SOD, GP, GR et CA) et/ou la détoxification d'électrophiles (GST, AR). Cette lignée est ainsi particulièrement adaptée à l'étude des phénomènes d'inflammations ou d'irritations de la cornée humaine.

La lignée cellulaire selon l'invention exprime aussi des marqueurs métaboliques de différenciation qui sont spécifiques des cellules de la cornée, notamment des cellules épithéliales de la cornée humaine (voir Reiners et al., Carcinogenesis, 11, 957-963, 1990). Ces marqueurs de différentiation peuvent être un ARNm, une protéine, ou une structure cellulaire différentiée.

La lignée selon l'invention, exprime au moins 2 marqueurs de différentiation choisis dans le groupe formé par la cytokératine de 64kD, la glutathion-S-transférase hGST5.8, et un profile en cytokines et en facteurs de croissance comprenant les composés TNFα, IL-1β, IL-1α, IL-6, IL-8, GM CSF-β, IL-ra, TGF-β1, TGF-β2, TGFα, EGF, PDGF-β.

La lignée selon l'invention est également capables d'exprimer au moins un marqueur spécifique d'une réaction inflammatoire, c'est à dire une molécule qui est détectable lorsque la cellule réagit à une stimulation inflammatoire et/ou une molécule ou un structure qui est nécessaire à une réaction inflammatoire.

Les stimulations inflammatoires peuvent être induites lorsque l'on met en contact les lignées selon l'invention avec un microorganisme de l'espèce *Pseudomonas aeruginosa* (Kernacki et al., J. Ocul. Pharmacol., 10, 281-288, 1994), avec le facteur d'activation des plaquettes PAF (Bazan et al., Exp. Eye Research, 14, 769-775, 1995), avec du 12-*O*-tetradecanoylphorbol (TPA: tumor promoting agent), avec l'histamine (Sharif et al., J. Ocular Pharmacol., 10, 653-664, 1994), avec la bradykinine (Sharif et al., Neurochem. Int., 18, 89-96, 1991), ou avec d'autres composés inflammatoires, par exemple.

La collagénase I est un marqueur facilement détectable lorsque une lignée est soumise à une stimulation inflammatoire (Bazan et al., Proc. Natl. Acad. Sci. USA, 90, 8678-8682, 1993). D'autres marqueurs peuvent aussi être détectés, comme le c-fos (voir Bazan et al.), les intermédiaires arachidoniques 12-HETE et 12-HETrE (Conners et al., Inves. Ophth. & Visu. Sci., 36, 828-840, 1995) ou un nouveau profile en cytokines et en facteurs de croissance, par exemple.

La lignée selon l'invention est également capable d'exprimer au moins un marqueur nécessaire à d'une réaction inflammatoire, comme par exemple le récepteur à la bradykinine, le récepteur à l'histamine, le récepteur au PAF, et le système de transduction d'un signal par les phosphoinositides (Phosphoinositide turnover signal transduction pathway; Sharif et al., Neurochem. Res., 12, 1313-1320, 1993).

L'invention concerne en particulier une lignée immortalisée selon l'invention qui a été déposée, sous le traité de Budapest, auprès de la Collection Nationale de Cultures de Microorganismes, Institut Pasteur, 25 rue du Docteur Roux, 75724 Paris, le 22 octobre 1996, où elle a reçue le numéro de dépôt CNCM I-1777.

La lignée épithéliale selon l'invention conserve ses caractéristiques de différentiation originelle même après de nombreux passages, notamment au moins 25 passages, de préférence au moins 30 à 100 passages.

Pour obtenir la lignée selon l'invention, on peut préparer une culture de cellules épithéliales primaires issues de la cornée humaine, infecter la culture avec un virus recombinant, et cultiver les cellules immortalisées dans un milieu de culture sans sérum. A cet effet l'homme du métier dispose de nombreux milieux de culture sans sérum. A titre d'indication, on peut citer les milieux sans sérum décrits par Gibson et al. (Gut, 35, 791-797, 1991), Pfeifer et al. (Proc. Natl. Acad. Sci. USA, 90, 5123-5127, 1993), Kulkani et al. (Carcinogenesis, 16, n°10, 2515-2521, 1995), ou dans EP96201064,1 et US08/576483, par exemple. Un milieu sans sérum parfaitement adapté au besoin de ce procédé est le milieu KGM (Clonetics, US).

La lignée selon l'invention a été obtenue en mettant en oeuvre les étapes suivantes:
(1) on obtient un échantillon de tissus épithéliaux d'une cornée humaine;
(2) on prépare cette échantillon en vue de sa culture *in-vitro*;
(3) on ensemence les cellules épithéliales dans un milieu de culture sans sérum et sur des plaques de culture comprenant un revêtement qui facilite l'attachement des cellules et leur croissance;
(4) on change le milieu autant de fois que nécessaire pour optimiser la croissance confluante;
(5) on infecte les cellules avec un virus recombinant;
(6) et on cultive les cellules immortalisées dans un milieu de culture sans sérum.

Plus en détail, l'étape 1) concerne l'obtention d'échantillons de cellules de la cornée d'individus normaux lors d'actes de chirurgie. A l'étape 2), on peut laver l'échantillon dans le milieu de culture, le couper en morceaux, séparer la partie épithéliales des autres tissus par des moyens physiques et/ou chimiques. Par exemple, on peut placer les morceaux de tissus dans une solution comprenant une protéase pendant un temps suffisant pour arriver à séparer les cellules.

A l'étape 3), on peut ensemencer les cellules épithéliales dans un milieu de culture sans sérum, les plaques de culture présentant un revêtement constitué d'une solution de gélatine à 0,01-1%, par exemple.

A l'étape 4), le milieu de culture contenant les cellules épithéliales est changé autant de fois que nécessaire pour optimiser une croissance confluante. De préférence, le milieu est remplacé tous les deux jours. Après avoir atteint une confluence de l'ordre de 90% de la surface disponible, ce qui intervient généralement 10 à 14 jours après l'ensemencement, les cellules sont séparées par des traitements dans une solution de protéases.

Les cellules séparées sont transférées à l'étape 5) dans un milieu de culture frais, puis les cellules sont ensuite infectées d'une manière classique par un virus recombinant. De nombreuses techniques de transfection sont à la disposition de l'homme du métier. A titre d'exemple on peut citer les techniques décrites dans WO96/07750, par Claudia Chen et al. (Mol. and Cell. Biol., 7, 2745-2752, 1987) ou par Wilson et al. (Analytical Biochemistry, 226, 212-220, 1995).

De préférence on utilise un virus recombinant SV40 comprenant l'Antigène T (Ag-T), une origine de réplication de virus inactivée, et un gène de sélection. A titre d'exemple, on peut utiliser la construction d'ADN pLXSHD+SV40+ décrite par Stockshlaeder et al dont la séquence est disponible dans la banque de donnée GeneBank (n° accession M64753; Human Gen. Therapy, 2, 33-39, 1991).

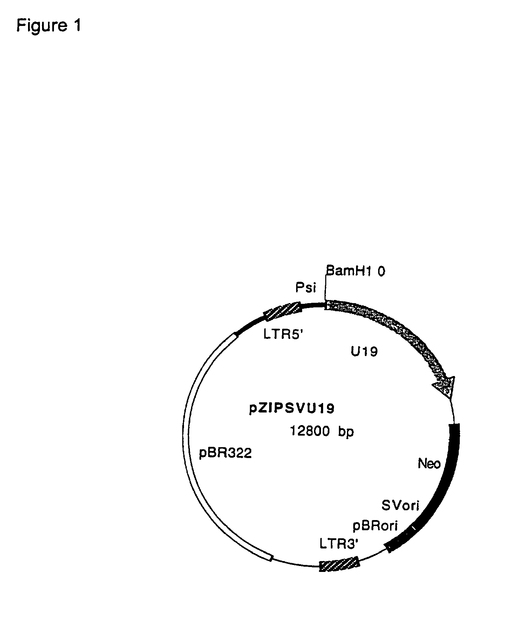

D'autres vecteurs appropriés peuvent être aussi facilement construits par l'homme du métier à partir de vecteurs disponibles commercialement comprenant le gène codant pour Ag-T, un gène de sélection et/ou une origine de réplication de virus inactivée. A titre d'exemple, on peut préparer la construction p1/SV40U19 en créant des sites Bam*HI* aux extrémités du fragment Bg*I*-Hpa*I* de SV40, et en clonant ce fragment dans le site Bam*HI* du plasmide pZIPSVU19 décrit à la figure 1 ci-après, et par Jat et al., Mol. Cell. Biol., 9, 1672-1681, 1989.

A l'étape 6) on transfert les cellules épithéliales dans un milieu de croissance frais, sur des plaques de culture ayant le revêtement décrit ci-dessus.

Connaissant les propriétés nouvelles et originales de la lignée de cellules épithéliales selon l'invention, on peut envisager son application dans des études immunologiques, pharmacologiques et toxicologiques

La lignée selon l'invention est ainsi particulièrement adaptée pour cribler des agents mutagéniques, toxiques ou bénéfiques pour le métabolisme des cellules de la cornée, par exemple dans un procédé dans lequel (1) on fait réagir, on cultive ou on met en contact avec une culture comprenant la lignée cellulaire selon l'invention, un agent soupçonné d'être un agent mutagène, toxique ou bénéfique pour le métabolisme des cellules de la cornée, et (2) on détermine ou on mesure les effets dudit agent sur ladite lignée cellulaire.

On peut donc envisager également de préparer un kit de diagnostique comprenant les cellules épithéliales selon l'invention, et des réactifs pour déterminer une réponse métabolique desdites cellules à des agents mutagéniques, toxiques ou bénéfiques.

La lignée selon l'invention est aussi adaptée à l'expression de protéines recombinantes. Les méthodes de transfection d'ADN étranger sont maintenant bien connues de l'homme du métier (voir par exemple WO 94/05472)

La lignée selon l'invention a aussi une utilité potentielle dans la thérapie génétique *ex-vivo.* Cette lignée pourrait constituer en effet un outil adéquat pour développer des cellules recombinantes exprimant des gènes d'intérêt en vue d'une application thérapeutique. Un avantage présenté en plus par la lignée selon l'invention, est qu'elle n'est pas exposée à un sérum animal, ce qui réduit considérablement les risques de contaminations potentiels par des virus ou autres agents pathogènes.

La présente invention est décrite plus en détail ci-après à l'aide du complément de description qui va suivre, qui se réfère à des exemples de préparations de la lignée cellulaire selon l'invention. La culture des lignées cellulaires, la préparation de vecteurs SV40, la transfection, la préparation des sondes et des amorces d'ADN, et l'analyse de l'expressions des marqueurs sont, en l'absence de précisions contraires, effectuées selon les protocoles décrits dans les références citées ci-dessus et dans ceux de l'ouvrage de Sambrook *et al*. (Molecular Cloning, A Laboratory Manual, Cold Spring Harbor Laboratory Press, U.S.A., 1989). Les pourcentages sont donnés en poids, sauf indication contraire.

### Exemple 1

On construit le plasmide p1/SV40U19 en créant des sites Bam*HI* aux extrémités du fragment Bg*I*-Hpa*I* de SV40, et en clonant ce fragment dans le site Bam*HI* du plasmide pZIPSVU19 décrit par Jat et al., Mol. Cell. Biol., 9, 1672-1681, 1989. A titre d'indication, le vecteur pZIPSVU19 représenté schématiquement à la figure 1, comprend le marqueur de sélection néomycine et le LTR du virus Moloney.

On prépare les virus SV40 selon une version modifiée du protocole de Lynch et Miller (J. Virol., 65, 3887-3890, 1991). Pour cela, on cultive les lignées cellulaires ecotropique Psi2 (Mann et al., Cell, 33, 153-159, 1983) et amphotropique PA317 (ATCC CRL9078) dans le milieu DMEM (Dulbecco, USA) comprenant 10% de serum foetal de veau, à 37°C et sous une atmosphère comprenant 5% de CO₂. On transfecte classiquement ces lignées séparément en utilisant 250mM de CaCl2 et 10 µg du plasmide pl/SV40U19, on les soumet à un traitement à la trypsine après 48h d'incubation, on les mélange en quantité égale, et on incube le tout à 37°C, sous une atmosphère comprenant 5% de CO₂. Après croissance jusqu'à 80% de confluence, on récolte les virus dans le milieu sans sérum KGM-1 (= milieu KBM de Clonetics, US, comprenant en outre 0,15 mM de CaCl, 30 µg/ml d'extrait de glande pituaire bovine, 0,5µg/ml d'hydrocortisone, 5 µg/ml d'insuline, 10 µg/ml de transférine, 10 ng/ml de facteur de croissance épidermal murin (EGF), 10000 U/ml de pénicilline et 10000 U/ml de Streptomycine). Après filtration (0,45 µm, Micropore), on détermine la quantité de virus sur des cellules NIH 3T3 (ATCC CRL 1658).

Des cellules primaires épithéliales de cornée ont été obtenues suite à la biopsie de l'oeil d'un donneur de 73 ans décédé d'un infarctus du myocarde. On lave l'échantillon dans le tampon phosphate PBS, on le traite à 4°C pendant 24h avec 10 U/ml de dispase II (Boehringer Mannheim, n°295825) dans un tampon comprenant 50% de milieu KGM-2 (= KBM + 0,05 mM de CaCl, 30 µg/ml d'extrait de glande pituaire bovine, 10 ng/ml de facteur de croissance épidermal murin, 0,5 µg/ml d'hydrocortisone, 0,05 µg/ml d'amphotericine B, 5 µg/ml d'insuline, 10 µg/ml de transférine, 50 µg/ml de gentamycine). Après incubation, on sépare manuellement les cellules, on les lave dans le milieu DMEM (Dulbecco's modified Eagle medium, US) comprenant 10% de sérum foetal bovin, on les centrifuge, on reprend les culots dans le milieu KGM-2, et on les étale sur des plaques de culture préalablement préincubées dans une solution de 0,1% de gélatine A (Sigma G2500). On incube les plaques à 37°C sous une atmosphère comprenant 100% d'humidité relative, 95% d'oxygène et 5% de dioxyde de carbone. Le milieu de culture contenant les cellules épithéliales est changé autant de fois que nécessaire pour optimiser une croissance confluante.

Après avoir atteint une confluence de l'ordre de 90% après 2 semaines de culture, les cellules sont séparées classiquement par traitements dans une solution de dispase II. Les cellules séparées sont ensuite transférées dans le milieu KGM-1 et dans des plaques de culture préincubées avec une solution de 0,1% de gélatine A. Les cultures sont ensuite infectées en présence de 8 µg/ml de polybrene et un haut titre de virus recombinant SV40 (10⁵-10⁷ CFU) préparé comme décrit ci-dessus. Après 2 h d'incubation avec le virus, on lave les cultures dans du PBS et on cultive les cellules par passages successifs dans du milieu frais KGM-1, en prenant garde à chaque passage de successivement laver les cellules dans un tampon HBSS (Hanks Balnced Salt Solution, Biofluids n°325); les séparer par un traitement pendant 1-2h à 37°C dans une solution comprenant 2,4 U/ml de dispase II, 50% de tampon HBSS et 50% de milieu KBM; les récolter dans du milieu KBM; les centrifuger; reprendre le culot dans le milieu KBM-1; puis étaler les cellules sur de nouvelles plaques fraîches.

Par passages successifs, on a purifié les cellules transformées des cellules épithéliales primaires. Puis en diluant, dans un milieu de culture, les cellules transformées préalablement séparées par la dispase II, on a pu sélectionner 9 lignées immortalisées individualisées de cellules épithéliales de la cornée humaine, dont une a été déposée, sous le traité de Budapest, auprès de la Collection Nationale de Cultures de Microorganismes, Institut Pasteur, 25 rue du Docteur Roux, 75724 Paris, le 22 octobre 1996, où elle a reçue le numéro de dépôt CNCM I-1777.

### 1. Analyse du caryotype de la souche CNCM I-1777

L'analyse du caryotype de la souche CNCM I-1777 a été faite par le "Children's Hospital of Michigan", 3901 Beaubien Blvd, Detroit, conformément à la méthode décrite dans le manuel "Human cytogenetics, Edts: Rooney DE, Czepulkowski BH, IRL Press, Oxford, 1986. Les résultats montrent que la lignée conserve intacte dans son génome un chromosome de chaque paire de chromosomes. Le sexe de la lignée est XX.

### 2. Tumorigenicité de la souche CNCM I-1777

On injecte 10⁷ cellules de la lignée CNCM I-1777 prise au 33^{ième} passage à des souris sans défense immunitaire ("nude") selon le protocole décrit par Stauffer et al. (The American journal of surgery, 169, 190-196, 1995). Aucune formation de tumeur n'est visible après plusieurs mois.

### 4. Contamination virale de la lignée CNCM I-1777

On détermine si la lignée CNCM I-1777 est contaminée par le virus de l'hépatite C (HCV), le virus de l'hépatite B (HBV) et le virus du sida (HIV-1). Les résultats des expériences décrites ci-après sont négatifs pour la présence des 3 virus.

Pour l'analyse du HBV, on extrait de l'ADN à partir d'environ 2x10⁶ cellules par traitement dans des solutions de phénol et chloroforme suivi d'une précipitation dans l'éthanol. Des échantillons d'ADN sont alors soumis à une amplification par PCR en utilisant des amorces spécifiques de la région pré-core du virus (Lynch et al., J. Virol., 65, 3887-3890). On sépare ensuite les produits de l'amplification sur gel agarose, et on les visualise sous U.V. en présence de bromure d'éthidium. Pour comparaison, on analyse de la même manière en parallèle une dilution d'un sérum contenant 10-10⁵ HBV (Anawa Biomedical Services 6 Product, USA).

Pour l'analyse du HIV-1, on soumet des échantillons d'ADN décrits ci-dessus soumis à une amplification par PCR en utilisant des amorces spécifiques de la région GAG du virus. On sépare ensuite les produits de l'amplification sur gel agarose, et on les visualise sous U.V. en présence de bromure d'éthidium. Pour comparaison, on analyse de la même manière en parallèle une dilution d'un sérum contenant 10-10⁵ HIV-1 (Anawa Biomedical Services 6 Product, USA).

Pour l'analyse du HCV, on extrait l'ARN à partir d'environ 2x10⁶ cellules par la méthode de Chomczynski et al. (Anal. Biochem., 162, 156-159, 1987). On effectue classiquement une réverse-transcription, et on soumet l'ADN complémentaire obtenu à une amplification par PCR en utilisant des amorces spécifiques de la région 5' non-codante du virus. On sépare ensuite les produits de l'amplification sur gel agarose, et on les visualise sous U.V. en présence de bromure d'éthidium. Pour comparaison, on analyse de la même manière en parallèle une dilution d'un sérum contenant 10-10⁵ HCV (Anawa Biomedical Services 6 Product, USA).

### 5. Marqueurs spécifiques de cellules épithéliales humaines pour la lignée CNCM I-1777

5.a. Cytokeratines: on fixe les cellules d'une culture de la lignée CNCM I-1777, prise au 22^{ième} passage, sur des plaques en verre par une solution de 100% méthanol froid, puis on lave les plaques dans un tampon comprenant 0,05M de Tris pH 8,6, 1,8% de NaCl et 0,2% de polyéthylène glycol 2000 (tampon TNP). On incube ensuite les cellules pendant 30 min en présence d'anticorps de souris spécifiques pour certaines cytokeratines (anti-CH peptide 4, 7, 8, 13, 14, 17, 18, 19, 20: Sigma et Boehringer). Après 3 lavages dans le tampon TNP comprenant 0,5% de BSA, on incube les plaques pendant 60 min avec un anticorps de chèvre anti IgG de souris comprenant un composé immunofluorescent (1:300, FITC-goat anti mouse IgG; Biosys). Après 3 lavages dans le tampon précédent, on fixe les plaques et on les analyse par fluorescence sous microscopie. Toutes les cellules sont positives pour les cytokeratines 4, 7, 8, 13, 14, 17, 18, 19.
5.b. Vimentine: on fixe les cellules d'une culture de la lignée CNCM I-1777, prise au 22^{ième} passage, comme décrit ci-dessus. Puis, on soumet les cellules fixées à un anticorps de souris anti-vimentine (DAKO, USA), puis à l'anticorps de chèvre anti-IgG de souris mentionné ci-dessus. Sous microscopie par fluorescence, toutes les cellules sont positives pour la vimentine.
5.c. Connections entre les cellules: on cultive sur plaque de verre jusqu'à confluence les cellules de la lignée CNCM I-1777, prise au 22^{ième} passage, on les fixe par traitement avec une solution de 2,5% de glutaraldéhyde dans un tampon phosphate 0,1 M pH 7,4 pendant 1 h, à température ambiante. Après deux lavages dans le même tampon phosphate, on fixe à nouveau les cellules dans une solution de 2% OsO₄ dans le même tampon. On déshydrate ensuite les cellules dans des solutions successives d'éthanol à 30, 50, 70, 90 et 100% (Polaron Equipment Ltd., Watford, UK), puis on recouvre les cellules par une fine couche d'or (SEM coating unit E5100, Polaron), On examine ensuite les cellules sous microscopie électronique (Philips 505 SEM). L'analyse révèle que les cellules présentent des connections inter-cellulaires qui sont caractéristiques des cellules épithéliales (en anglais "tight junctions").
5.d. Cytochrome P450: On analyse l'expression des cytochromes par les cellules de la lignée CNCM 1-1777, prise au 30^{ième} passage, à l'aide de la technique connue RT-PCR en utilisant des amorces d'ADN spécifiques des différentes cytochromes P450. Ces amorces ont été préparées classiquement à partir des séquences d'ADN des différentes cytochromes qui sont accessibles sur la base de donnée GeneBank (CYP1A1: n° accession X02612; CYP2C: n° accession M61855 ou M61858 ou M61856 ou MM61854; CYP2D6: n° accession M33388; CYP1A2: n° accession Z00036).
   Pour cela, on cultive les cellules jusqu'à confluence sur des boites de 35 mm (Costar), on extrait l'ARN à l'aide du kit RNAeasy (Qiagen), on effectue une reverse transcription (1st Strand cDNA Synthesis Kit for RT-PCR, Boehringer Mannheim), on soumet l'ADN complémentaire obtenu à une amplification par PCR en utilisant les amorces d'ADN spécifiques des différentes cytochromes P450, on sépare ensuite les produits de l'amplification sur gel d'agarose, et on les visualise sous U.V. en présence de bromure d'éthidium. Les résultats montrent que les cellules CNCM I-1777 expriment les cytochromes CPY1A1, CYP1A2, CYP2C, CYP2D6.
5.e. Enzymes impliquées dans l'oxydation cellulaire et la détoxification d'électrophiles: on cultive les cellules de la lignée CNCM I-1777 prise au 30^{ième} passage, on extrait l'ARN par la méthode de Chomczynski et al. (Anal. Biochem., 162, 156-159, 1987), puis on effectue un Nothern-blot de cet ARN avec des sondes d'ADN codant partiellement pour la Cu/Zn-superoxide dismutase (SOD), la glutathion péroxidase (GP), la catalase (CA) et la glutathion-S-transférase π (GSTπ). Les sondes d'ADN sont préparées classiquement à partir des séquences d'ADN des gènes codant pour ces enzymes qui sont disponibles sur la base de donnée GeneBank (GSTπ: n°accession X08058; GP: n°accession M21304; CA: n°accession M81578; SOD: n°accession 729336). Les résultats de ces essais montrent que toutes les cellules expriment une transcription des gènes codant pour l'activité SOD, GP, CA, et GSTπ.

Par ailleurs, l'expression de la glutathion réductase est également confirmée par le test enzymatique décrit par Gondhowiardjo (Cornea, 12, 310-314, 1993). L'activité catalase est aussi confirmée par le test enzymatique décrit par Aeby et al. (Method and Enzymology, 105, 121-126, 1984). Enfin, l'activité aldéhyde réductase est aussi mise en évidence par le test enzymatique décrit par Ellis et al. (Biochemical Journal, 312, 535-541, 1995).

### 6. Marqueurs spécifiques des cellules épithéliales de la cornée pour la lignée CNCM I-1777

6.a. Expression de la cytokératine de 64kD: comme pour le l'analyse des cytokératines décrite ci-dessus, on fixe les cellules sur plaques en verre par une solution de 100% méthanol froid, puis on lave les plaques dans le tampon TNP (voir ci-dessus). On incube ensuite les cellules pendant 30 min en présence d'anticorps de souris spécifiques pour la cytokératine de 64kD (Anticorps AE5, ICM Biomedical Inc., US). Après 3 lavages dans le tampon TNP comprenant 0,5% de BSA, on incube les plaques pendant 30 min avec un anticorps de chèvre anti IgG de souris comprenant un composé immunofluorescent (1:300, FITC-goat anti mouse IgG; Biosys). Après 3 lavages dans le tampon précédent, on fixe les plaques et on les analyse par fluorescence sous microscopie. Les cellules sont positives pour la cytokératine de 64 kD.
6.b. Expression de la glutathion-S-transférase hGST5.8: on analyse l'expression de la glutathion-S-transférase hGST5.8 au moyen du test décrit par Singhal et al. (Invest. Ophthalmol. Vis. Sci., 36, 142-150, 1995). En résumé, on mélange 10 µg/ml d'un extrait cytosolique de la lignée CNCM I-1777 avec 0,1 mM de 4-hydroxy-nonenal et 0,5 mM de glutathion dans un tampon phosphate de potassium 100 mM à pH 6,5, puis on mesure l'activité enzymatique par le changement d'absorption du mélange à 224 nm. Les résultats montrent que la lignée CNCM I-1777 exprime une activité glutathion-S-transférase hGST5.8.
6.c. Profile d'expression des cytokines et des facteurs de croissance: on cultive les cellules de la lignée CNCM I-1777 prise au 30^{ième} passage, on extrait l'ARN, puis on effectue une PCR sur cet ARN avec différentes amorces pour confirmer la transcription des ARN codant pour les cytokines et/ou les facteurs de croissance suivants: TNFα, IL-1β, IL-1α, IL-6, IL-8, GM CSF-β, IL-ra, TGF-β1, TGF-β2, TGFα, EGF et PDGF-β.

Ces amorces correspondent à une partie des gènes codant pour ces composés, les séquences d'ADN desdits gènes étant accessibles sur la base de donnée GeneBank (n° accession TNFα: X02910; IL-1β: M15840; IL-1α: M15329; IL-6: M14584, Rosenbaum et al., Inv. Opth. & Vi. Sc., 36, 2151-2155, 1995; IL-8: M28130; GM CSF-β: X03021; IL-ra: M97748, Kennedy et al., J. Clinical Inv., 95, 82-88, 1995; TGF-β1: X02812, Nishida et al., Curr. Eye Res., 14, 235-241, 1995; TGF-β2: Y00083; TGFα: M22440; EGF: L17032; PDGF-β: X02811).

Pour comparaison, on effectue la même analyse du profile d'expression des cytokines et des facteurs de croissance, sur de l'ARN provenant de tissus épithéliaux de la cornée humaine (4 biopsies différentes).

Les résultats sont présentés dans le tableau 1 ci-après. Le sigle * indique que l'expression des composés IL-1β, TNFα, IL-1α, IL-6 et IL-8, a été également confirmée par des tests ELISA à partir du milieu de culture des cellules CNCM I-1777. Les anticorps spécifiques contre ces différentes cytokines sont disponibles dans le commerce (IL-1β, TNFα: BioSource Inter., US, n° catalogue KHC0012 et KHC3013; IL-1α, IL-6 et IL-8: CLB corp., US, n° catalogue M1901, M1916, M1918).

**Tableau 1**

| Cytokines & Facteurs de croissance | CNCM I-1777 | Biopsie 1 | Biopsie 2 | Biopsie 3 | Biopsie 4 |
|---|---|---|---|---|---|
| TNFα* | + | +/- | +/- | +/- | +/- |
| IL-1β * | +++ | + | ++ | + | ++ |
| IL-1α* | +++ | +/- | ++ | + | +++ |
| IL-6 * | ++ | + | + | - | - |
| IL-8 * | +++ | +/- | + | + | ++ |
| GM CSF-β | ++ | +/- | +/- | +/- | +/- |
| IL-ra | ++ | + | +++ | ++ | + |
| TGF-β1 | +++ | + | ++ | + | ++ |
| TGF-β2 | +++ | + | ++ | ++ | ++ |
| TGFα | +++ | + | ++ | + | ++ |
| EGF | +++ | + | ++ | ++ | +++ |
| PDGF-β | + | +/- | +/- | +/- | +/- |

| | | | | | |
|---|---|---|---|---|---|
| (-: non détecté; +/-: détecté faiblement; +++: abondant) | | | | | |

### 7. Marqueurs spécifiques d'une réaction inflammatoire pour la lignée CNCM I-1777

7.a. Collagénase I: la collagénase I est une molécule dont l'expression caractérise un état normal d'inflammation des cellules de la cornée. On peut détecter l'expression de la collagénase par PCR, par Western-Blot ou par Elisa.
   Pour détecter la collagénase I par PCR, on cultive les cellules CNCM I-1777, prises au 29 ^{ième} passage, en présence de 20 ng/ml de TPA pendant 16 à 24h, on extrait l'ARN, et on détecte la présence d'ARNm de collagénase I par PCR avec une amorce dérivée de la séquence d'ADN de la collagénase I (Genebank: n°X05231).
   Pour détecter la collagénase I par Western-Blot, après avoir cultivé les cellules CNCM I-1777 en présence de 20 ng/ml de TPA pendant 16 à 24h, on récupère le milieu de culture, on le concentre avec un filtre Amicon30®, on sépare les protéine par électrophorèse sur un gel de polyacrylamide SDS-Page, on les transfert par Western-blot sur un filtre, on soumet le filtre à un premier anticorps anti-collagénase I (Cortex Biochem, US, n° 60338P), à un deuxième anticorps couplé à une péroxydase (Pierce, US, n° 31400), puis au substrat de la péroxydase.
   Pour détecter la collagénase I par Elisa, il suffit d'utiliser le kit hMMP-1 d'Amershan (catalogue, rpn 2610), en suivant les recommandations du fournisseur.
   Les résultats montrent que l'on peut détecter l'expression de collagénase I lorsque les cellules CNCM I-1777 sont soumises à un traitement inflammatoire. Par contre lorsque les cellules CNCM I-1777 ne sont pas mises en contact avec du TPA, celles-ci n'expriment pas de collagénase I détectable par PCR, Western-Blot ou Elisa.
7.b. Marqueurs nécessaires à une réaction inflammatoire: les récepteurs à la bradykinine, à l'histamine, au PAF, et le système de transduction des phosphoinositides sont des marqueurs indispensables pour que la lignée CNCM I-1777 puisse être physiologiquement dans un état normal d'inflammation. Pour détecter la présence de ces marqueurs, on incube les cellules CNCM I-1777 ou des cellules épithéliales primaires de la cornée humaine, en présence de composés inflammatoires qui sont supposés interagir avec des récepteurs couplés avec le système de transduction de signaux inflammatoires par les phosphoinositides, et on mesure la concentration de molécules de phosphate d'inositol (PI) ainsi produits. L'accumulation de PI est en fait caractéristique de l'existence de récepteurs aux composés inflammatoires.

Les essais sont basés sur les protocoles de Sharifs et al. décrits dans J. Ocular Pharmacol., 10, 653-664, 1994; et Neurochem Res., 12, 1313-1320, 1993. En résumé, sur des plaques comprenant 24 puits, on cultive dans le milieu DMEM environ 2,5x10⁵ cellules CNCM I-1777 par puis, en présence de [³H] myo-inositol (1µCi/0,5 ml; 15-17 Ci/mM, Amersham Corp.), pendant 24h à 37°C. On aspire le milieu des puits, et on les expose pendant 60 min à 37°C à du milieu DMEM comprenant 10mM de LiCl, 15 mM de tampon HEPES, et différentes concentrations de bradykinine (Collaborative Biomedical, US), d'histamine (Sigma US) ou de PAF (BioMol Research Lab, US). Pour arrêter la réaction, on aspire ensuite le milieu, et on ajoute dans les puits 0,1M d'acide formique. Après cela, on lyse les cellules, on sépare les composés du lysat sur une colonne d'échange d'ions (résine AG1x8; colonne Econo®, Biorad, US) en éluant la colonne avec 10ml d'eau déionisé et 8 ml d'une solution de 50mM de formate d'ammonium, et on quantifie la radioactivité dégagée par les fractions d'élution avec un compteur à scintillation.

Les résultats sont analysés selon la méthode décrite par Sharif et al. (Neurochem. Int., 18, 89-96, 1991). La valeur EC50 définit la concentration de composé requise pour stimuler 50% du maximum d'activité du système de transduction des phosphoinositides.

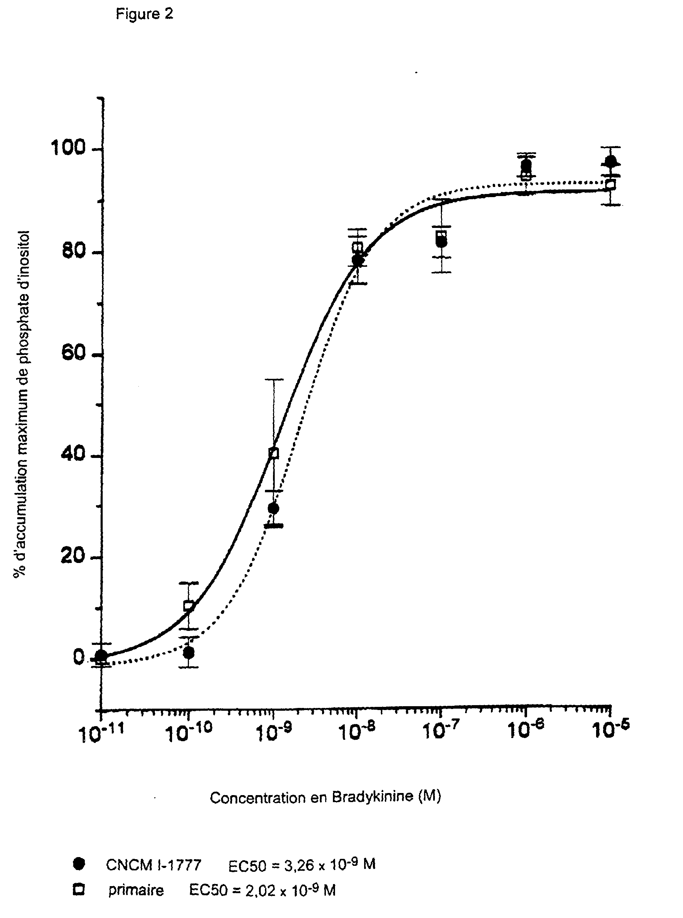

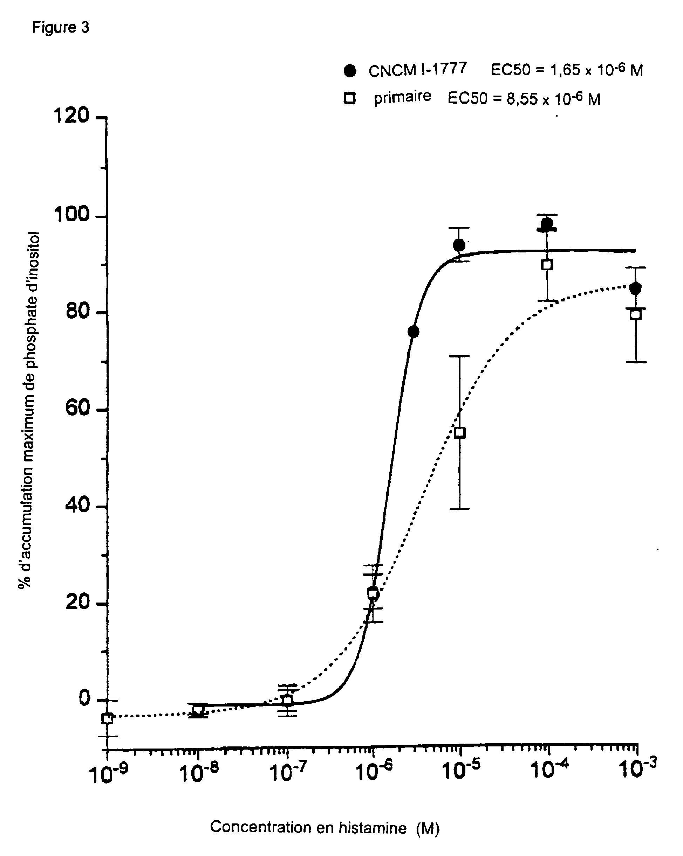

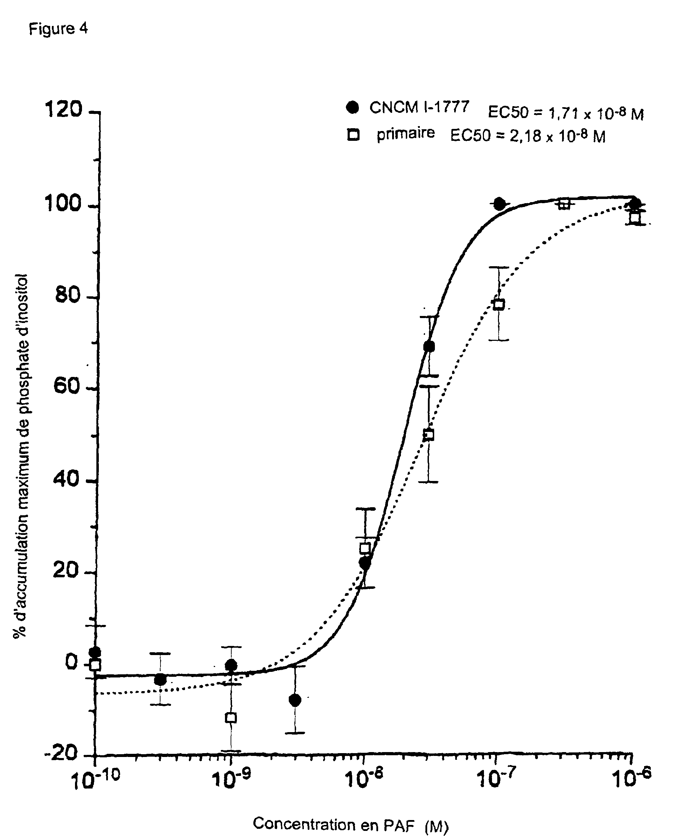

Les résultats, présentés aux figures 2, 3 et 4, montrent que les cellules CNCM I-1777 présentent des récepteurs à la bradykinine, à l'histamine, au PAF, et qu'elles se comportent comme des cellules épithéliales primaires de cornée humaine.

### Exemple 2

Comme décrit à l'exemple 1, on analyse la capacité de la lignée CNCM I-1777 prise au passage 106 à exprimer des marqueurs métaboliques spécifiques des cellules épithéliales humaines non-immortalisées, des marqueurs métaboliques de différenciation spécifiques des cellules épithéliales non-immortalisées de la cornée humaine, et des marqueurs d'une réaction inflammatoire. Les résultats sont comparables à ceux présentés à l'exemple 1.

## Revendications

1. Lignée immortalisée de cellules épithéliales humaines de cornée capable de se stratifier, et capable d'exprimer des marqueurs métaboliques spécifiques des cellules épithéliales humaines non-immortalisées, des marqueurs métaboliques de différenciation spécifiques des cellules épithéliales non-immortalisées de la cornée humaine, et des marqueurs spécifiques d'une réaction inflammatoire, ladite lignée immortalisée présentant le numéro de dépôt CNCM I-1777.

2. Procédé pour identifier l'effet mutagène, toxique ou bénéfique d'un agent sur le métabolisme des cellules de la cornée, dans lequel (1) on fait réagir une culture comprenant une lignée cellulaire selon la revendication 1 avec un agent soupçonné d'être un agent mutagène, toxique ou bénéfique pour le métabolisme des cellules de la cornée humaine, et (2) on détermine les effets dudit agent sur ladite lignée cellulaire.

3. Kit de diagnostique comprenant les cellules épithéliales immortalisées de la cornée humaine selon la revendication 1, et des réactifs pour déterminer une réponse métaboliques desdites cellules à des agents mutagéniques, toxiques ou bénéfiques.

4. Utilisation des cellules épithéliales immortalisées de la cornée humaine selon la revendication 1, dans des procédés d'identification d'agents mutagéniques, toxiques ou bénéfiques pour le métabolisme des cellules de la cornée.

5. Utilisation des cellules épithéliales immortalisées de la cornée humaine selon la revendication 1, en tant qu'agent pharmaceutique actif.

## Claims

1. Immortalised human corneal epithelial cell line capable of stratification and capable of expressing specific metabolic markers of non-immortalised human epithelial cells, specific metabolic differentiation markers of non-immortalised human corneal epithelial cells, and specific markers of an inflammatory reaction, said immortalised line having deposit number CNCM I-1777.

2. Method for identifying the mutagenic, toxic or beneficial effect of an agent on the metabolism of corneal cells, in which (1) a culture comprising a cell line according to claim 1 is reacted with an agent suspected of being a mutagenic, toxic or beneficial agent for the metabolism of human corneal cells, and (2) the effects of said agent on said cell line are determined.

3. Diagnostic kit comprising the immortalised human corneal epithelial cells according to claim 1 and reagents for determining a metabolic response of said cells to mutagenic, toxic or beneficial agents.

4. Use of the immortalised human corneal epithelial cells according to claim 1 in methods for identifying mutagenic, toxic or beneficial agents for the metabolism of corneal cells.

5. Use of the immortalised human corneal epithelial cells according to claim 1 as a pharmaceutical active agent.

## Patentansprüche

1. Immortalisierte humane Epithelzelllinie der Cornea, die die Fähigkeit besitzt, sich in Schichten anzuordnen, Stoffwechechselmarker zu exprimieren, die spezifisch sind für nicht-immortalisierte humane Epithelzellen, Stoffwechselmarker zu exprimieren, die spezifisch sind für differenzierte humane Epithelzellen der Cornea, sowie Marker zu exprimieren, die für eine Entzündungsreaktion spezifisch sind, wobei die besagte immortalisierte Zellinie die Hinterlegungsnummer CNCM I-1777 aufweist.

2. Verfahren zur Identifizierung von mutagenen, toxischen oder für den Stoffwechsel der Zellen der Cornea nutzbringenden Stoffen, wobei man (1) eine Kultur, die die Zelllinie gemäss Anspruch 1 umfasst, mit einem Stoff reagieren lässt, von dem man annimmt, dass er mutagen, toxisch oder für den Stoffwechsel der Zellen der humanen Cornea nutzbringend ist, und wobei man (2) die Auswirkungen des besagten Stoffes auf die besagte Zelllinie ermittelt.

3. Diagnosekit, der die immortalisierten humanen Epithelzellen der Cornea gemäß Anspruch 1 sowie Reagenzien umfasst, die der Ermittelung einer Stoffwechselantwort besagter Zellen auf mutagene, toxische oder für besagte Zellen nutzbringende Stoffe dienen.

4. Verwendung der immortalisierten humanen Epithelzellen der Cornea gemäß Anspruch 1 in Verfahren zur Identifikation von mutagenen, toxischen oder für den Stoffwechsel der Zellen der Cornea nutzbringenden Stoffen.

5. Verwendung der immortalisierten humanen Epithelzelllen der Cornea gemäß Anspruch 1 als pharmazeutischer Wirkstoff.
